# EUROPEAN PATENT APPLICATION

(11) **EP 1 967 201 A1**
(43) Date of publication of application: **10.09.2008**
(21) Application number: 06833077.8
(22) Date of filing: 21.11.2006
(51) Int. Cl.: A61K 38/00, A61L 27/00, A61P 17/02, C07K 14/575, C12N 15/00

(54) **THERAPEUTIC AGENT FOR SKIN OR SKIN REPAIR-PROMOTING AGENT COMPRISING DES-ACYLGHRELIN OR DERIVATIVE THEREOF AS ACTIVE INGREDIENT**

(30) Priority: 21.11.2005 JP 2005336420
(71) Applicant: The University of Miyazaki, Miyazaki-shi, Miyazaki 889-2192 (JP); Japan as represented by president of National Cardiovascular Center, Suita-shi, Osaka 565-8565 (JP); Asubio Pharma Co., Ltd., Minato-ku Tokyo 107-8541 (JP)
(72) Inventor: MURAKAMI, Noboru, Miyazaki-shi Miyazaki 889-2192 (JP); NAKAHARA, Keiko, Miyazaki-shi Miyazaki 889-2192 (JP); KANGAWA, Kenji, Suita-shi, Osaka 565-8565 (JP); HAYASHI, Yujiro, Ohra-gun, Gunma 370-0503 (JP)
(74) Representative: Findeisen, Marco
(86) International application number: PCT/JP2006/323231
(87) International publication number: WO 2007/058360

(57) **Abstract**

The present invention provides a novel therapeutic agent for skin injuries and a skin regeneration accelerator. The therapeutic agent for skin injuries and the skin regeneration accelerator containing desacyl ghrelin or a pharmaceutically acceptable salt thereof as an active ingredient.

SEQUENCE LISTING

<110>  University of Miyazaki
       National Cardiovascular Center, President
       Daiichi Asubio Pharma.Co.,Ltd.

<120>   An agent for promoting skin restoration or treating skin damage
which contains, as an active ingredient, des-acyl ghrelin or its deriva-
tive.

<130>  D05F2173

<160>  21

<210>  1
<211>  28
<212>  PRT
<213>  Homo sapiens

<220>
<221>  PEPTIDE
<222>  (1)..(28)
<223>  Amino acid sequence for human endogenous peptides of growth hormo-
       ne secretagogue receptor

<400>  1
Gly Ser Ser Phe Leu Ser Pro Glu His Gln Arg Val Gln Gln Arg Lys
1               5                   10                  15
Glu Ser Lys Lys Pro Pro Ala Lys Leu Gln Pro Arg
            20                  25

<210>  2
<211>  27
<212>  PRT
<213>  Homo sapiens

<220>
<221>  PEPTIDE
<222>  (1)..(27)
<223>  Amino acid sequence for human endogenous peptides (27 amino acids

) of growth hormone secretagogue receptor

<400> 2
Gly Ser Ser Phe Leu Ser Pro Glu His Gln Arg Val Gln Arg Lys Glu
1               5                   10                  15
Ser Lys Lys Pro Pro Ala Lys Leu Gln Pro Arg
            20                  25

<210> 3
<211> 28
<212> PRT
<213> Rattus norvegicus

<220>
<221> PEPTIDE
<222> (1)..(28)
<223> Amino acid sequence for rat endogenous peptides of growth hormone
secretagogue receptor

<400> 3
Gly Ser Ser Phe Leu Ser Pro Glu His Gln Lys Ala Gln Gln Arg Lys
1               5                   10                  15
Glu Ser Lys Lys Pro Pro Ala Lys Leu Gln Pro Arg
            20                  25

<210> 4
<211> 27
<212> PRT
<213> Rattus norvegicus

<220>
<221> PEPTIDE
<222> (1)..(27)
<223> Amiino acid sequence for rat endogenous peptides of growth hormon-
e secretagogue receptor

<400> 4
Gly Ser Ser Phe Leu Ser Pro Glu His Gln Lys Ala Gln Arg Lys Glu
1               5                   10                  15

Ser Lys Lys Pro Pro Ala Lys Leu Gln Pro Arg
            20                  25

<210> 5
<211> 28
<212> PRT
<213> Mus musculus

<220>
<221> PEPTIDE
<222> (1)..(28)
<223> Amino acid sequence for mouse endogenous peptides of growth hormo-
ne secretagogue receptor

<400> 5
Gly Ser Ser Phe Leu Ser Pro Glu His Gln Lys Ala Gln Gln Arg Lys
1               5                   10                  15
Glu Ser Lys Lys Pro Pro Ala Lys Leu Gln Pro Arg
            20                  25

<210> 6
<211> 28
<212> PRT
<213> Sus scrofa

<220>
<221> PEPTIDE
<222> (1)..(28)
<223> Amino acid sequence for porcine endogenous peptides of growth hor-
mone secretagogue receptor

<400> 6
Gly Ser Ser Phe Leu Ser Pro Glu His Gln Lys Val Gln Gln Arg Lys
1               5                   10                  15
Glu Ser Lys Lys Pro Ala Ala Lys Leu Lys Pro Arg
            20                  25

<210> 7

<211>  27
<212>  PRT
<213>  Bos taurus

<220>
<221>  PEPTIDE
<222>  (1)..(27)
<223>  Amino acid sequence for bovine endogenous peptides (27 amino acid-
       s) of growth hormone secretagogue receptor

<400>  7
Gly Ser Ser Phe Leu Ser Pro Glu His Gln Lys Leu Gln Arg Lys Glu
1               5                   10                  15
Ala Lys Lys Pro Ser Gly Arg Leu Lys Pro Arg
            20                  25

<210>  8
<211>  27
<212>  PRT
<213>  Ovis aries

<220>
<221>  PEPTIDE
<222>  (1)..(27)
<223>  Amino acid sequence for ovine endogenous peptides (27 amino acids
       ) of growth hormone secretagogue receptor

<400>  8
Gly Ser Ser Phe Leu Ser Pro Glu His Gln Lys Leu Gln Arg Lys Glu
1               5                   10                  15
Pro Lys Lys Pro Ser Gly Arg Leu Lys Pro Arg
            20                  25

<210>  9
<211>  28
<212>  PRT
<213>  Canis familiaris

<220>

<221>  PEPTIDE
<222>  (1)..(28)
<223>  Amino acid sequence for dog endogenous peptides of growth hormone
        secretagogue receptor

<400>  9
Gly Ser Ser Phe Leu Ser Pro Glu His Gln Lys Leu Gln Gln Arg Lys
1               5                   10                  15
Glu Ser Lys Lys Pro Pro Ala Lys Leu Gln Pro Arg
            20                  25

<210>  10
<211>  21
<212>  PRT
<213>  Anguilla japonica

<220>
<221>  PEPTIDE
<222>  (1)..(21)
<223>  Amino acid sequence for eel endogenous peptides of growth hormone
        secretagogue receptor. This peptide is amidated at C-terminus.

<400>  10
Gly Ser Ser Phe Leu Ser Pro Ser Gln Arg Pro Gln Gly Lys Asp Lys
1               5                   10                  15
Lys Pro Pro Arg Val
            20

<210>  11
<211>  23
<212>  PRT
<213>  Oncorhynchus mykiss

<220>
<221>  PEPTIDE
<222>  (1)..(23)
<223>  Amino acid sequence for rainbow trout endogenous peptides (23 ami-
       no acids) of growth hormone secretagogue receptor. This peptide i-
       s amidated at C-terminus.

<400>  11

Gly Ser Ser Phe Leu Ser Pro Ser Gln Lys Pro Gln Val Arg Gln Gly
1               5                   10                  15

Lys Gly Lys Pro Pro Arg Val
            20

<210>  12
<211>  20
<212>  PRT
<213>  Oncorhynchus mykiss

<220>
<221>  PEPTIDE
<222>  (1)..(20)
<223>  Amino acid sequence for rainbow trout endogenous peptides (20 ami-
       no acids) of growth hormone secretagogue receptor.  This peptide
       is amidated at C-terminus.

<400>  12
Gly Ser Ser Phe Leu Ser Pro Ser Gln Lys Pro Gln Gly Lys Gly Lys
1               5                   10                  15

Pro Pro Arg Val
            20

<210>  13
<211>  24
<212>  PRT
<213>  Gallus domesticus

<220>
<221>  PEPTIDE
<222>  (1)..(24)
<223>  Amino acid sequence for chicken endogenous peptides of growth hor-
       mone secretagogue receptor

<400>  13

Gly Ser Ser Phe Leu Ser Pro Thr Tyr Lys Asn Ile Gln Gln Gln Lys

1                   5                   10                  15

Gly Thr Arg Lys Pro Thr Ala Arg
            20

<210>  14
<211>  24
<212>  PRT
<213>  Gallus domesticus

<220>
<221>  PEPTIDE
<222>  (1)..(24)
<223>  Amino acid sequence for chicken endogenous peptides of growth hor-
       mone secretagogue receptor

<400>  14
Gly Ser Ser Phe Leu Ser Pro Thr Tyr Lys Asn Ile Gln Gln Gln Lys
1               5                   10                  15
Asp Thr Arg Lys Pro Thr Ala Arg
            20

<210>  15
<211>  26
<212>  PRT
<213>  Gallus domesticus

<220>
<221>  PEPTIDE
<222>  (1)..(26)
<223>  Amino acid sequence for chicken endogenous peptides of growth hor-
       mone secretagogue receptor

<400>  15
Gly Ser Ser Phe Leu Ser Pro Thr Tyr Lys Asn Ile Gln Gln Gln Lys
1               5                   10                  15
Asp Thr Arg Lys Pro Thr Ala Arg Leu His
            20                  25

<210> 16
<211> 27
<212> PRT
<213> Rana catesbeiana

<220>
<221> PEPTIDE
<222> (1)..(27)
<223> Amino acid sequence for frog endogenous peptides of growth hormon-
e secretagogue receptor

<400> 16
Gly Leu Thr Phe Leu Ser Pro Ala Asp Met Gln Lys Ile Ala Glu Arg
1               5                   10                  15
Gln Ser Gln Asn Lys Leu Arg His Gly Asn Met
            20                  25

<210> 17
<211> 28
<212> PRT
<213> Rana catesbeiana

<220>
<221> PEPTIDE
<222> (1)..(28)
<223> Amino acid sequence for frog endogenous peptides of growth hormon-
e secretagogue receptor

<400> 17
Gly Leu Thr Phe Leu Ser Pro Ala Asp Met Gln Lys Ile Ala Glu Arg
1               5                   10                  15
Gln Ser Gln Asn Lys Leu Arg His Gly Asn Met Asn
            20                  25

<210> 18
<211> 20
<212> PRT
<213> Tilapia nilotica

<220>

<221>  PEPTIDE

<222>  (1)..(20)

<223>  Amino acid sequence for tilapia endogenous peptides of growth hor-
       mone secretagogue receptor.  This peptide is amidated at C-termin-
       us.

<400>  18

Gly Ser Ser Phe Leu Ser Pro Ser Gln Lys Pro Gln Asn Lys Val Lys
1               5                   10                  15

Ser Ser Arg Ile
            20

<210>  19

<211>  22

<212>  PRT

<213>  Silurus asotus

<220>

<221>  PEPTIDE

<222>  (1)..(22)

<223>  Amino acid sequence for tilapia endogenous peptides of growth hor-
       mone secretagogue receptor.  This peptide is amidated at C-termin-
       us.

<400>  19
Gly Ser Ser Phe Leu Ser Pro Thr Gln Lys Pro Gln Asn Arg Gly Asp
1               5                   10                  15

Arg Lys Pro Pro Arg Val
            20

<210>  20

<211>  23

<212>  PRT

<213>  Silurus asotus

<220>

<221>  PEPTIDE

<222>  (1)..(23)
<223>  Amino acid sequence for catfish endogenous peptides of growth hor-
       mone secretagogue receptor

<400>  20
Gly Ser Ser Phe Leu Ser Pro Thr Gln Lys Pro Gln Asn Arg Gly Asp
1               5                   10                  15
Arg Lys Pro Pro Arg Val Gly
            20

<210>  21
<211>  28
<212>  PRT
<213>  Equus caballus

<220>
<221>  PEPTIDE
<222>  (1)..(28)
<223>  Amino acid sequence for equine endogenous peptides of growth horm-
       one secretagogue receptor

<400>  21
Gly Ser Ser Phe Leu Ser Pro Glu His His Lys Val Gln His Arg Lys
1               5                   10                  15
Glu Ser Lys Lys Pro Pro Ala Lys Leu Lys Pro Arg
            20                  25

<210>  22
<211>  15
<212>  PRT
<213>  Rattus norvegicus

<220>
<221>  PEPTIDE
<222>  (1)..(15)
<223>  Amino acid sequence (1-15 amino acids) derived from rat endogenous
       peptides of growth hormone secretagogue receptor. This peptide is
       amidated at C-terminus.

<400> 22
Gly Ser Ser Phe Leu Ser Pro Glu His Gln Lys Ala Gln Gln Arg
1               5                   10                  15

## Description

### TECHNICAL FIELD

The present invention relates to skin cell proliferation activity of a ghrelin-related substance. More specifically, the present invention relates to a therapeutic agent or composition for skin injuries, a skin regeneration accelerator and a method for culturing a skin cell in skin injury treatment where the substance is used as an active ingredient.

### BACKGROUND ART

It is obvious, without looking back at the long history of skin transplantation, that skin is a tissue with which transplantation therapy has been attempted from the earliest time. Further, looking ahead to the future study on transplantation and regeneration, skin is considered to be the tissue that allows the easiest access and whose development/regeneration process has been thoroughly verified, through skin development studies, regeneration studies using hair, and so on. In addition, the skin transplantation and regeneration is an area of study which can be directly applied to various skin diseases, such as heat injuries, epidermolysis bullosa hereditaria and male pattern alopecia, and is expected to play an important role in skin injuries or skin lesions (see the website of Kyoto University 21st Century Center of Excellence Program "Establishment of International COE for Integration of Transplantation Therapy and Regenerative Medicine"- Study on Skin Transplantation and Regeneration, http://www.kuhp.kyoto-u.ac.jp/∼coe/member15.html).

Cultured skin to be needed in skin transplantation differs depending on the disease, the extent of injury and the transplantation area. While transplantation of a cultured epidermis alone is appropriate in the case of moderate or lighter burn injuries, cultured skin incorporating a dermal layer is necessary for a patient suffering from full thickness skin damage. It is desired to develop cultured skin that works as wound dressing and potentially releases a physiologically active substance for the purpose of treating intractable skin ulcers in patients with diseases including diabetes (see Takamura: Bio Venture, 1:58 (2001)).

Currently, there are two kinds of cultured skin sheets used for skin transplantation: one is normal epidermal keratinocyte according to a method developed by Green et al. (see Rheinwald and Green: Cell, 6:331 (1975)); and the other is skin equivalent tissue developed by Bell et al. (see Bell et al.: Science, 211: 1052 (1981)). Green et al. used 3T3 cells as supporting cells to suppress the differentiation of epidermal keratinocyte, and successfully gained sufficient proliferation. According to the method, the epidermal keratinocyte is multilayered during culture to form a sheet-like structure, and the cultured cell structure similar to living structure has been shown to be clinically useful as a skin tissue replacement. Using a material such as collagen, Bell et al. preliminarily prepared a part corresponding to dermis, and cultured an epidermal keratinocyte thereon. In this method, it was essential to use a material contributing to three-dimensional incorporation of cells for artificial tissue formation, and collagen gel was a proper material for the purpose (see Hata: Jikken Igaku Extra Number, 19: 2121 (2001)).

Once a scaffold for cells is prepared, the next necessary thing is a substance that promotes proliferation and differentiation of a cell (see Ueda: Bio Venture, 1: 32 (2001)). People cautious about clinical application of IGF, TGF-β, FGF, or the like insist that it may cause abnormality in differentiation and development of cells in other parts of a human body. Therefore, it is desired to discover/develop a highly safe substance with this activity.

In the transplantation therapy or regenerative medicine for injured skin, future application of a cultured fetal skin cell is also desired because a cultured fetal skin cell transplanted to a injured region differentiates and proliferates into normal skin, that is, has a skin repair ability (see Hohfeld et al.: Lancet, 366: 788-790 (2005)). However, since the proliferation speed of the fetal skin cell is slow, developing a technology to promote the proliferation is desired.

Ghrelin is a hormone found in the stomach in 1999. It is a peptide having an amino acid sequence composed of 28 residues and having an extremely rare chemical structure in which a third amino acid from the N terminus in the sequence is acylated with fatty acid (see Kojima et at.: Nature, 402, 656-660 (1999), and see WO01/07475). Ghrelin acts on a growth hormone secretagogue-receptor 1a (GHS-R1a, see Haward et al.: Science 273: 974-977 (1996)), and is described as an endogenous cerebral-gastrointestinal hormone that promotes secretion of a growth hormone (GH) from pituitary (see Kojima et at.: Nature, 402, 656-660 (1999)). Recent studies have revealed that ghrelin increases appetite, that subcutaneous administration of ghrelin increases body weight and body fat, and that ghrelin has activities such as improving cardiac function (see Wren et at.: Endocrinology 141: 4325-4328 (2000), Nakazato et al.: Nature 409: 194-198 (2001), Shintani et al.: Diabetes 50: 227-232 (2001), Lely et al.: Endocr. Rev. 25: 426-457 (2004), and Korbonits et al.: Front Neuroendocrino. 25: 27-68 (2004)).

Since ghrelin has GH secretion promoting activity and appetite stimulation activity, it is expected that ghrelin thereby further effectively extracts fat-burning activity for converting fat into energy, and effect of strengthening muscles through the anabolic activity that GH expresses (see Akamizu and Kangawa: Saishin Igaku, 60: 1569-1573 (2005)).

Ghrelin was first isolated from a rat, and purified as an endogenous GHS acting on GHS-R1a. Since then, amino acid sequences of ghrelin having a similar primary structure have been found from various vertebrates other than rat, such as human, mouse, porcine, chicken, eel, bovine, equine, ovine, bullfrog, trout and canine.

### Human

:GSS(n-occtanoyl)FLSPEHQRVQQRKESKKPPAKLQPR (SEQ ID NO: 1 )
:GSS(n-octanoyl)FLSPEHQRVQRKESKKPPAKLQPR (SEQ ID NO: 2 ) Rat
:GSS(n-octanoyl)FLSPEHQKAQQRKESKKPPAKLQPR (SEQ ID NO: 3 )
:GSS(n-octanoyl)FLSPEHQKAQRKESKKPPAKLQPR (SEQ ID NO:4 ) Mouse
:GSS(n-octanoyl)FLSPEHQKAQQRKESKKPPAKLQPR (SEQ ID NO: 5 ) Porcine
:GSS(n-octanoyl)FLSPEHQKVQQRKESKKPAAKLKPR (SEQ ID NO: 6 ) Bovine
:GSS(n-octanoyl)FLSPEHQKLQRKEAKKPSGRLKPR (SEQ ID NO: 7 ) Ovine
:GSS(n-octanoyl)FLSPEHQKLQRKEPKKPSGRLKPR (SEQ ID NO: 8 ) Canine
:GSS(n-octanoyl)FLSPEHQKLQQRKESKKPPAKLQPR (SEQ ID NO: 9 ) Eel
:GSS(n-octanoyl)FLSPSQRPQGKDKKPPRV-NH₂ (SEQ ID NO: 10 ) Trout
:GSS(n-octanoyl)FLSPSQKPQVRQGKGKPPRV-NH₂ (SEQ ID NO: 11 )
:GSS(n-octanoyl)FLSPSQKPQGKGKPPRV-NH₂ (SEQ ID NO: 12 ) Chicken
:GSS(n-octanoyl)FLSPTYKNIQQQKGTRKPTAR (SEQ ID NO: 13 )
:GSS(n-octanoyl)FLSPTYKNIQQQKDTRKPTAR (SEQ ID NO: 14 )
:GSS(n-octanoyl)FLSPTYKNIQQQKDTRKPTARLH (SEQ ID NO: 15 ) Bullfrog
:GLT(n-octanoyl)FLSPADMQKIAERQSQNKLRHGNM (SEQ ID NO: 16 )
:GLT(n-decanoyl)FLSPADMQKIAERQSQNKLRHGNM (SEQ ID NO: 16 )
:GLT(n-octanoyl)FLSPADMQKIAERQSQNKLRHGNMN (SEQ ID NO: 17 ) Tilapia
:GSS(n-octanoyl)FLSPSQKPQNKVKSSRI-NH₂ (SEQ ID NO: 18 ) Catfish
:GSS(n-octanoyl)FLSPTQKPQNRGDRKPPRV-NH₂ (SEQ ID NO: 19 )
:GSS(n-octanoyl)FLSPTQKPQNRGDRKPPRVG (SEQ ID NO: 20 ) Equine
:GSS(n-butanoyl)FLSPEHHKVQHRESKKPPAKLKPR (SEQ ID NO: 21 )
(In the above expression, an amino acid residue is represented by the single character expression).

The above peptide has a specific structure, wherein a side chain hydroxyl group of a serine residue (S) or a threonine residue (T) at a third position is acylated with fatty acid such as octanoic acid and decanoic acid. Except for ghrelin, there has been no case of isolation of a physiologically active peptide having a hydrophobic modification structure.

A desacyl form (desacyl ghrelin) has been reported as a metabolite that is produced by decomposition of an acyl group in a modified site of ghrelin. It is known with Activities such as suppression of apoptosis of a myocardial cell and suppression of lipid decomposition in an adipocyte (see Baldanzi et al. J. Cell Biol., 195: 1029-1037 (2002) and Muccioli et al. Eur. J. Pharmacol., 498: 27-35 (2004)).

As far as the inventors know, no report has been found yet regarding any activity of desacyl ghrelin on skin cells and proliferation of skin cells by the activity of the peptide on skin cells.

### DISCLOSURE OF THE INVENTION

### PROBLEM TO BE SOLVED BY THE INVENTION

The present invention relates to a therapeutic agent for skin injuries, a skin regeneration accelerator and a method for culturing a skin cell in skin injury treatment by the use of a substance having skin cell proliferation activity.

### MEANS FOR SOLVING THE PROBLEM

The present inventors have found that desacyl ghrelin, which is reportedly a metabolite produced by decomposition of an acyl group (such as an octanoyl group of a serine residue in the third position) in a modified site of ghrelin, exists in amniotic fluid of a pregnant rat and fetal blood. Considering the function and role of desacyl ghrelin played in amniotic fluid and blood of the fetus, it is expected that desacyl ghrelin has a fetal skin cell proliferation activity.

The present inventors have studied how desacyl ghrelin acts on skin cells, and found that desacyl ghrelin receptor exists in the skin cells, that desacyl ghrelin acts on the skin cell to increase intracellular calcium, and that desacyl ghrelin expressed the proliferation activity of the skin cell.

That is, the present invention relates to a skin regeneration accelerator in skin injury treatment, a formation accelerator for a cultured skin cell sheet made by culturing a skin cell, and a skin repair accelerator and a therapeutic agent upon transplantation of the cultured skin, containing desacyl ghrelin as an active ingredient.

The present invention also relates to a skin injury treating method, a method for accelerating the formation of a cultured skin cell sheet made by culturing a skin cell, and a method for accelerating skin repair upon transplantation of the cultured skin as well as a method for treating the above disease by the repair acceleration, comprising administration of desacyl ghrelin.

Further, the present invention relates to the use of desacyl ghrelin for a skin regeneration accelerator in skin injury treatment, an accelerator for formation of a cultured skin cell sheet made by culturing a skin cell, and producing a skin repair accelerator and a therapeutic agent upon transplantation of the cultured skin.

Based on the above description, the present invention specifically relates to the following matters.
(1) A therapeutic agent for skin injuries, comprising desacyl ghrelin or a peptide having physiological activity that is substantially identical to that of desacyl ghrelin, or a pharmaceutically acceptable salt thereof, as an active ingredient.
(2) The therapeutic agent according to (1), wherein the active ingredient is a peptide selected from the group consisting of (i) a peptide having an amino acid sequence represented by SEQ ID NO: 1 to SEQ ID NO: 21, (ii) a peptide having the amino acid sequence from the N terminus to the 4^{th} amino acid residue of an amino acid sequence represented by SEQ ID NO: 1 to SEQ ID NO: 21, and having the amino acid sequence from the 5^{th} amino acid residue to the C terminus wherein one or several amino acids are deleted, substituted and/or added, (iii) peptides of (1) and (2), in which the C terminus is amidated, or a pharmaceutically acceptable salt thereof.
(3) The therapeutic agent according to (2), wherein the active ingredient is a peptide having an amino acid sequence represented by SEQ ID NO: 1 or a pharmaceutically acceptable salt thereof.
(4) The therapeutic agent according to (2), wherein the active ingredient is a peptide having the amino acid sequence from the N terminus to the 15^{th} amino acid residue of an amino acid sequence represented by SEQ ID NO: 1, of which the 15^{th} amino acid is amidated, or a pharmaceutically acceptable salt thereof.
(5) The therapeutic agent according to any one of (1) to (4), including the active ingredient in an amount of 0.001 mg to 100 mg per unit formulation.
(6) A skin regeneration accelerator, comprising desacyl ghrelin or a peptide having physiological activity that is substantially identical to that of desacyl ghrelin, or a pharmaceutically acceptable salt thereof, as an active ingredient.
(7) The skin regeneration accelerator according to (6), wherein the active ingredient is a peptide selected from the group consisting of (i) a peptide having an amino acid sequence represented by SEQ ID NO: 1 to SEQ ID NO: 21, (ii) a peptide having the amino acid sequence from the N terminus to the 4^{th} amino acid residue of an amino acid sequence represented by SEQ ID NO: 1 to SEQ ID NO: 21, and having the amino acid sequence from the 5^{th} amino acid residue to the C terminus wherein one or several amino acids are deleted, substituted and/or added, (iii) peptides of (1) and (2), in which the C terminus is amidated, or a pharmaceutically acceptable salt thereof.
(8) The skin regeneration accelerator according to (7), wherein the active ingredient is a peptide having an amino acid sequence represented by SEQ ID NO: 1 or a pharmaceutically acceptable salt thereof.
(9) The skin regeneration accelerator according to (7), wherein the active ingredient is a peptide having the amino acid sequence from the N terminus to the 15^{th} amino acid residue of an amino acid sequence represented by SEQ ID NO: 1, of which the 15^{th} amino acid is amidated, or a pharmaceutically acceptable salt thereof.
(10) The skin regeneration accelerator according to any one of (6) to (9), including the active ingredient in an amount of 0.001 mg to 100 mg per unit formulation.
(11) A method for accelerating proliferation of a cultured skin cell, using desacyl ghrelin or a peptide having physiological activity that is substantially identical to that of desacyl ghrelin, or a pharmaceutically acceptable salt thereof, as an active ingredient.
(12) The method according to (11), wherein the active ingredient is a peptide selected from the group consisting of (i) a peptide having an amino acid sequence represented by SEQ ID NO: 1 to SEQ ID NO: 21, (ii) a peptide having the amino acid sequence from the N terminus to the 4^{th} amino acid residue of an amino acid sequence represented by SEQ ID NO: 1 to SEQ ID NO: 21, and having the amino acid sequence from the 5^{th} amino acid residue to the C terminus wherein one or several amino acids are deleted, substituted and/or added, (iii) peptides of (1) and (2), in which the C terminus is amidated, or a pharmaceutically acceptable salt thereof.
(13) The method according to (12), wherein the active ingredient is a peptide having an amino acid sequence represented by SEQ ID NO: 1 or a pharmaceutically acceptable salt thereof.
(14) The method according to (12), wherein the active ingredient is a peptide having the amino acid sequence from the N terminus to the 15^{th} amino acid residue of an amino acid sequence represented by SEQ ID NO: 1, of which the 15^{th} amino acid is amidated, or a pharmaceutically acceptable salt thereof.
(15)The method according to any one of (11) to (14), wherein proliferation of the cultured skin cell is performed in a medium containing the active ingredient in an amount of 0.0000001 mg/mL to 0.1 mg/mL.
(16) A treatment method of skin injuries, comprising administration of desacyl ghrelin or a peptide having physiological activity that is substantially identical to that of desacyl ghrelin, or a pharmaceutically acceptable salt thereof as an active ingredient to a mammal requiring treatment of skin injuries.
(17) The method according to (16), wherein the active ingredient is a peptide selected from the group consisting of (i) a peptide having an amino acid sequence represented by SEQ ID NO: 1 to SEQ ID NO: 21, (ii) a peptide having the amino acid sequence from the N terminus to the 4^{th} amino acid residue of an amino acid sequence represented by SEQ ID NO: 1 to SEQ ID NO: 21, and having the amino acid sequence from the 5^{th} amino acid residue to the C terminus wherein one or several amino acids are deleted, substituted and/or added, (iii) peptides of (1) and (2), in which the C terminus is amidated, or a pharmaceutically acceptable salt thereof.
(18) The method according to (17), wherein the active ingredient is a peptide having an amino acid sequence represented by SEQ ID NO: 1 or a pharmaceutically acceptable salt thereof.
(19) The method according to (17), wherein the active ingredient is a peptide having the amino acid sequence from the N terminus to the 15^{th} amino acid residue of an amino acid sequence represented by SEQ ID NO: 1, of which the 15^{th} amino acid is amidated, or a pharmaceutically acceptable salt thereof.
(20) The method according to any one of (16) to (19), wherein the active ingredient is administered in an amount of 0.001 mg to 100 mg per administration.
(21) A method for accelerating skin regeneration, comprising administration of desacyl ghrelin or a peptide having physiological activity that is substantially identical to that of desacyl ghrelin, or a pharmaceutically acceptable salt thereof as an active ingredient to a mammal requiring skin regeneration.
(22) The method according to (21), wherein the active ingredient is a peptide selected from the group consisting of (i) a peptide having an amino acid sequence represented by SEQ ID NO: 1 to SEQ ID NO: 21, (ii) a peptide having the amino acid sequence from the N terminus to the 4^{th} amino acid residue of an amino acid sequence represented by SEQ ID NO: 1 to SEQ ID NO: 21, and having the amino acid sequence from the 5^{th} amino acid residue to the C terminus wherein one or several amino acids are deleted, substituted and/or added, (iii) peptides of (1) and (2), in which the C terminus is amidated, or a pharmaceutically acceptable salt thereof.
(23)The method according to (22), wherein the active ingredient is a peptide having an amino acid sequence represented by SEQ ID NO: 1 or a pharmaceutically acceptable salt thereof.
(24)The method according to (22), wherein the active ingredient is a peptide having the amino acid sequence from the N terminus to the 15^{th} amino acid residue of an amino acid sequence represented by SEQ ID NO: 1, of which the 15^{th} amino acid is amidated, or a pharmaceutically acceptable salt thereof.
(25)The method according to any one of (21) to (24), wherein the active ingredient is administered in an amount of 0.001 mg to 100 mg per administration.
(26) Use of desacyl ghrelin or a peptide having physiological activity that is substantially identical to that of desacyl ghrelin, or a pharmaceutically acceptable salt thereof as an active ingredient for production of a therapeutic agent for skin injuries.
(27)The use according to (26), wherein the active ingredient is a peptide selected from the group consisting of (i) a peptide having an amino acid sequence represented by SEQ ID NO: 1 to SEQ ID NO: 21, (ii) a peptide having the amino acid sequence from the N terminus to the 4^{th} amino acid residue of an amino acid sequence represented by SEQ ID NO: 1 to SEQ ID NO: 21, and having the amino acid sequence from the 5^{th} amino acid residue to the C terminus wherein one or several amino acids are deleted, substituted and/or added, (iii) peptides of (1) and (2), in which the C terminus is amidated, or a pharmaceutically acceptable salt thereof.
(28)The use according to (27), wherein the active ingredient is a peptide having an amino acid sequence represented by SEQ ID NO: 1 or a pharmaceutically acceptable salt thereof.
(29)The use according to (27), wherein the active ingredient is a peptide having the amino acid sequence from the N terminus to the 15^{th} amino acid residue of an amino acid sequence represented by SEQ ID NO: 1, of which the 15^{th} amino acid is amidated, or a pharmaceutically acceptable salt thereof.
(30)The use according to any one of (26) to (29), wherein the therapeutic agent for skin injuries contains the active ingredient in an amount of 0.001 mg to 100 mg per unit formulation.
(31)Use of desacyl ghrelin, or a peptide having a physiological action that is substantially identical to that of desacyl ghrelin, or a pharmaceutically acceptable salt thereof as an active ingredient for production of a skin regeneration accelerator.
(32)The use according to (31), wherein the active ingredient is a peptide selected from the group consisting of (i) a peptide having an amino acid sequence represented by SEQ ID NO: 1 to SEQ ID NO: 21, (ii) a peptide having the amino acid sequence from the N terminus to the 4^{th} amino acid residue of an amino acid sequence represented by SEQ ID NO: 1 to SEQ ID NO: 21, and having the amino acid sequence from the 5^{th} amino acid residue to the C terminus wherein one or several amino acids are deleted, substituted and/or added, (iii) peptides of (1) and (2), in which the C terminus is amidated, or a pharmaceutically acceptable salt thereof.
(33)The use according to (32), wherein the active ingredient is a peptide having an amino acid sequence represented by SEQ ID NO: 1 or a pharmaceutically acceptable salt thereof.
(34)The use according to (32), wherein the active ingredient is a peptide having the amino acid sequence from the N terminus to the 15^{th} amino acid residue of an amino acid sequence represented by SEQ ID NO: 1, of which the 15^{th} amino acid is amidated, or a pharmaceutically acceptable salt thereof.
(35)The use according to any one of (31) to (34), wherein the skin regeneration accelerator contains the active ingredient in an amount of 0.001 mg to 100 mg per unit formulation.

### ADVANTAGEOUS EFFECT OF THE INVENTION

The present invention reveals that desacyl ghrelin has skin cell proliferation activity. Therefore, the invention enables prompt application of cultured skin cells to treatment by adding the substance so as to accelerate the cell proliferation in culturing skin cells. That is, more quick supply of an artificial skin sheet made up of cultured skin cells to a patient becomes possible in the process of skin repair treatment in which a skin cell is cultured to prepare a sheet-shaped artificial skin and therewith an injured skin surface is covered. Further, healing acceleration becomes possible by transplanting skin cells directly to the site of a laceration or wound followed by administering desacyl ghrelin to the transplantation site.

Further, the present inventors have discovered that a previously unknown desacyl ghrelin receptor exists in a skin cell. The finding made it possible to screen for a substance acting on the desacyl ghrelin receptor using a fetal skin cell and to prepare a desacyl ghrelin receptor antibody using the cell.

### BRIEF DESCRIPTION OF THE DRAWINGS

Fig. 1 is a view showing the activity of desacyl ghrelin to increase intracellular calcium in a single cultured fetal skin cell.
Fig. 2 is a view showing the proliferation activity of desacyl ghrelin and desacyl ghrelin (1-15)-NH₂ in a cultured fetal skin cell.

### BEST MODE FOR CARRYING OUT THE INVENTION

The medicine of the present invention can be used as that for mammals (individual organisms) including human. Examples of the substance used in the present invention include desacyl ghrelin not having the acyl group in the modified site (such as an octanoyl group of a serine residue in the third position) of ghrelin.

As the desacyl ghrelin, as described above, a peptide having the same amino acid sequence as ghrelin derived from human, as well as ghrelin derived from a rat, mouse, porcine, bovine and other animals, and derivatives thereof can be used.

Preferably, for an individual organism, desacyl ghrelin derived from the same individual organism is used. For example, desacyl ghrelin having the same amino acid sequence as that of ghrelin derived from human is preferably used for a human body. The desacyl ghrelin is a peptide comprising 28 amino acids (SEQ ID NO: 1).

The derivatives of desacyl ghrelin include a peptide selected from the group consisting of (a) a peptide having an amino acid sequence from the N terminus to the 4^{th} amino acid residue of an amino acid sequence represented by SEQ ID NO: 1 to SEQ ID NO: 21, and having the amino acid sequence from the 5^{th} amino acid residue to the C terminus wherein one or several amino acids are deleted, substituted and/or added, and (b) a peptide having an amino acid sequence represented by SEQ ID NO: 1 to SEQ ID NO: 21, and a peptide of (a), in which the C terminus is amidated, the peptide having physiological activity that is substantially identical to that of desacyl ghrelin. Among these, a preferred peptide is, for example, a peptide having an amino acid sequence in which one or several amino acids are substituted, added and/or deleted in 5^{th} to 28^{th} amino acid residues from the amino terminus of an amino acid sequence represented by SEQ ID NO: 1, and having an activity of increasing the intracellular calcium ion concentration. Preferred examples of the amino acid residue to be added include basic amino acids, particularly lysine. It is desirable that the homology of the amino acid sequence of the derivatives is 70%, preferably 80%, more preferably 90%, further preferably 95%, and most preferably by 97%, as compared with the natural amino acid sequence. These conditions are the same in the desacyl ghrelin having the same amino acid sequence as that of ghrelin derived from other animals (SEQ ID NO: 2 to SEQ ID NO: 22). The derivatives the C terminus thereof is amidated are also included in the desacyl ghrelin derivatives of the present invention.

The desacyl ghrelin and the derivatives of the present invention may be prepared by a conventional method. For example, they may be isolated from natural raw materials, or produced by recombinant DNA technology and/or chemical synthesis. When modification (acylation) is necessary in an amino acid residue, modification reaction may be applied according to known means. For example, in the production method using recombinant DNA technology, the desacyl ghrelin according to the present invention and derivatives thereof can be obtained by culturing a host cell transformed by an expression vector having DNA that encodes a peptide according to the present invention and then by collecting the objective peptide from the culture.

Examples of the vector that incorporates a gene include E. coli vectors (such as pBR322, pUC18 and pUC19), bacillus subtilis vectors (such as pUB110, pTP5 and pC194), yeast vectors (such as YEp, YRp and YIp), and animal cell vectors (such as retrovirus and vaccinia virus). Any other vectors may be used, as long as they stably retain the objective gene in the host cell. The vector is introduced into a proper host cell. As a method for incorporating the objective gene into plasmid, or introducing it into the host cell, a method described in Molecular Cloning (Sambrook et al., 1989) may be used, for example.

In order to allow the objective peptide gene to express in the plasmid, a promoter is connected upstream of the gene in such a way that it functions.

The promoter used in the present application may be any promoter, as long as it is appropriate for the host cell used for expression of the objective gene. For example, when the host cell to be transformed is the genus Escherichia, a lac promoter, trp promoter, lpp promoter, λPL promoter, recA promoter or the like may be used; when the host cell is the genus Bacillus, a SPO1 promoter SPO2 promoter or the like may be used; when the host cell is a yeast, a GAP promoter, PHOS promoter, ADH promoter or the like may be used; and when the host cell is an animal cell, a SV40-derived promoter, retrovirus-derived promoter or the like may be used.

The host cell is transformed using the above-obtained vector containing the objective gene. The host cell to be used may be bacteria (such as genus Escherichia and genus Bacillus), yeasts (such as genus Saccharomyces, genus Pichia and genus Candida), and animal cells (such as CHO cell and COS cell). A liquid medium is appropriate as the culture medium, and the medium particularly preferably contains carbon and nitrogen sources which are necessary for the growth of a transformed cell to be cultured. Vitamins, growth secretagogue, serum, or the like may be added if desired.

After culture, the peptide according to the present invention is separated from the culture and purified by a conventional method. For example, extraction of the objective substance from cultured bacterial forms or cells is performed by collecting the bacterial forms or cells after culture, suspending it in a buffer solution containing a protein modifier (such as guanidine hydrochloride), crushing the bacterial forms or cells with ultrasound or the like, and centrifuging it. In order to purify the objective substance from the supernatant fluid, several separation and purification methods such as gel permeation, ultrafiltration, dialysis, SDS-PAGE and various chromatography techniques may be employed in proper combination according to the molecular weight, solubility, electric charge (isoelectric point) and affinity of the objective substance.

The desacyl ghrelin and the derivatives thereof according to the present invention can be chemically synthesized by a conventional method. For example, the substance may be prepared by condensation of amino acid with protecting groups by a liquid phase method and/or solid phase method, extending a peptide chain, removing the entire protecting groups with acid, and purifying the resultant crude product by the above purification method.

In regard to the production method of peptide, various methods have already been known. The peptide according to the present invention can also be easily prepared by a known method, for example, a classical peptide synthetic method or a solid phase method.

As the salts of desacyl ghrelin and the derivatives thereof used for the present invention, pharmaceutically acceptable salts are preferable. Examples thereof include a salt of an inorganic base, a salt of an organic base, a salt of an inorganic acid, a salt of an organic acid, a salt of basic amino acid and a salt of acidic amino acid.

Preferred examples of the salts of an inorganic base include alkali metal salts such as a sodium salt and a potassium salt; alkaline earth metal salts such as a calcium salt and a magnesium salt; an aluminum salt; an ammonium salt; and the like.

Preferred examples of the salts of an organic base include trimethylamine, triethylamine, pyridine, picoline, ethanolamine, diethanolamine, triethanolamine, dicyclohexylamine, N,N'-dibenzylethylenediamine, and the like.

Preferred examples of the salts of inorganic acid include salts of hydrochloric acid, hydrobromic acid, nitric acid, sulfuric acid, phosphoric acid, and the like.

Preferred examples of the salts of organic acid include salts of formic acid, acetic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid, and the like.

Preferred examples of the salts of basic amino acid include salts of arginine, lysine, ornithine, and the like while preferred examples of the salts of acidic amino acid include salts of aspartic acid, glutamic acid, and the like.

Among the above salts, a sodium salt and a potassium salt are most preferred.

In regard to the physiological activity of desacyl ghrelin, it is possible to select the derivatives using the intracellular calcium increasing activity as an indicator, with the skin cell of rat fetus in which desacyl ghrelin receptor has been discovered. As the measurement method for intracellular calcium ion concentration, a known method may be used. For example, FLIPR (Fluorometric Imaging Plate Reader by Molecular Devices Co., Ltd.) utilizing the fluctuation of fluorescence intensity of Fluo-4 AM (by Molecular Probe Co., Ltd.) resulting from the fluctuation of calcium ion concentration may be employed (Kojima et al.: Nature, 402: 656-660 (1999)). Also, to confirm whether or not the peptide having a calcium increasing activity has a desacyl ghrelin activity in vitro or in vivo, a known method may be used. For example, the in vitro activity can be measured by an ejection experiment in a binding assay, after binding [¹²⁵I]-labeled desacyl ghrelin to the skin cell of rat fetus. In order to confirm the desacyl ghrelin activity in vivo, apoptosis of a myocardial cell, or lipid concentration in adipose tissue or in serum is measured after injecting a peptide having a calcium increasing activity into a peripheral vein of an animal, or calcium concentration in a fetal skin is measured. In regard to the desacyl ghrelin derivatives and preparation method thereof, for example, J. Med. Chem., 43, pp.4370-4376, 2000 can be referred to.

The desacyl ghrelin according to the present invention has been found to have a skin cell proliferation activity and markedly increase the number of fetal skin cells in particular.

That is, desacyl ghrelin according to the present invention may be used as an active ingredient of a skin regeneration accelerator in skin injury treatment (such as wound, abrasion and burn), of a formation accelerator for a cultured skin cell sheet made by culturing a skin (such as epidermis, corium and skin)cell, and of a skin repair accelerator and a therapeutic agent upon transplantation of the cultured skin in burn injury, intractable skin ulcer, epidermolysis bullosa hereditaria, bed sore, obese cicatrix, birthmark, serious allergic skin disease and alopecia.

Further, administration of desacyl ghrelin according to the present invention to an individual organism is used in the method for skin injury treatment (such as wound, abrasion and burn), the method for formulation acceleration of a cultured skin cell sheet made by culturing a skin (such as epidermis, corium and skin)cell, the method for skin repair acceleration upon transplantation of the cultured skin in burn injury, intractable skin ulcer, epidermolysis bullosa hereditaria, bed sore, obese cicatrix, birthmark, serious allergic skin disease and alopecia, and the method for treating the above-mentioned diseases by the repair acceleration.

Further, desacyl ghrelin according to the present invention is used for production of a skin regeneration accelerator in skin injury treatment (such as wound, abrasion and burn), of a formation accelerator for a cultured skin cell sheet made by culturing a skin (such as epidermis, corium and skin)cell, and of a skin repair accelerator and a therapeutic agent upon transplantation of the cultured skin in burn injury, intractable skin ulcer, epidermolysis bullosa hereditaria, bed sore, obese cicatrix, birthmark, serious allergic skin disease and alopecia.

The drug of the present invention that contains desacyl ghrelin or the pharmacologically acceptable salt thereof may be used for individual organisms (such as a human, mouse, rat, rabbit, canine, feline, bovine, equine, porcine and primate) by mixing it with a pharmacologically acceptable carrier, excipient, extender or the like.

The drug of the present invention is preferably administered in single or multiple doses of a predetermined amount by a parenteral route such as intravenous, hypodermic and intramuscular injection to an individual undergoing skin regeneration treatment. When the individual is a human adult particularly under domiciliary treatment, transnasal, transpulmonary or suppository administration is preferred.

In the present invention, the dosage of the drug is not particularly limited, and may be properly selected depending on the intended use, and the age, body weight, kind of the individual, symptom, nutritional status and concomitant drugs of the individual to be administered. When the drug is administered in single or multiple doses to a human adult, the quantity of desacyl ghrelin as an active ingredient is preferably 0.001 mg to 100 mg, more preferably 0.01 mg to 10 mg.

It is preferred to administer the above dosage once or several times a day for a period of 1 to 24 weeks, more preferably 1 to 12 weeks.

Examples of the pharmaceutically acceptable carrier include diverse organic or inorganic carrier substances that are commonly used as materials for drug formulation. Such a carrier is blended as an excipient, lubricant, binder or disintegrant for a solid formulation; and a solvent, solubilizing agent, suspending agent, tonicity agent, buffer agent and soothing agent for a liquid formulation.

Formulation additives such as antiseptic, antioxidant, colorant and sweetener may be used if desired.

Preferred examples of the excipient include lactose, sucrose, D-mannitol, starch, crystalline cellulose, light anhydrous silicic acid, and the like.

Preferred examples of the lubricant include magnesium stearate, calcium stearate, talc, colloidal silica, and the like.

Preferred examples of the binder include crystalline cellulose, sucrose, D-mannitol, dextrin, hydroxypropyl cellulose, hydroxypropylmethyl cellulose, polyvinyl pyrrolidone, and the like.

Preferred examples of the disintegrant include starch, carboxymethyl cellulose, carboxymethyl cellulose calcium, croscarmellose sodium, carboxymethyl starch sodium, and the like.

Preferred examples of the solvent include an injection solvent, alcohol, propylene glycol, macrogol, sesame oil, corn oil, and the like.

Preferred examples of the solubilizing agent include polyethylene glycol, propylene glycol, D-mannitol, benzyl benzoate, ethanol, trisaminomethane, cholesterol, triethanolamine, sodium carbonate, sodium citrate, and the like.

Preferred examples of the suspending agent include surfactants such as stearyl triethanolamine, sodium lauryl sulfate, laurylaminopropionic acid, lecithin, benzalkonium chlorides, benzethonium chlorides and glycerin monostearate; hydrophilic polymers such as polyvinyl alcohol, polyvinyl pyrrolidone, carboxymethyl cellulose sodium, methylcellulose, hydroxymethyl cellulose, hydroxyethyl cellulose and hydroxypropyl cellulose; and the like.

Preferred examples of the tonicity agent include sodium chloride, glycerin, D-mannitol, and the like.

Preferred examples of the buffer agent include a phosphate, acetate, carbonate, citrate, and the like.

A preferred example of the soothing agent includes benzyl alcohol, and the like.

Preferred examples of the antiseptic include paraoxybenzoate esters, chlorobutanol, benzyl alcohol, phenetyl alcohol, dehydroacetic acid, sorbic acid, and the like.

Preferred examples of the antioxidant include a sulfite, ascorbic acid, and the like.

A preferred form of the drug of the present invention is a formulation suitable for parenteral administration. Examples of the formulation form suitable for parenteral administration include an injectable solution for intravenous, intradermal, hypodermic or intramuscular administration; an intravenous fluid; suppository; percutaneous absorbent; transmucosal absorbent and inhalant. Among the forms, the form of an injectable solution is preferred, and particularly when the individual is a human adult under domiciliary treatment, forms of a transmucosal absorbent, inhalant and suppository are preferred. Since these formulation forms are known to those skilled in the art, it is possible for them to select a formulation form suitable for the intended administration route and to formulate a drug in the form of a pharmaceutical composition using one or more additives usable in the pharmaceutical industry if desired.

For example, a medicine in the form of an injectable solution or intravenous fluid is prepared and provided by dissolving the substance acting on desacyl ghrelin as an active ingredient, together with one or more additives for formulation use such as a buffer solution, sugar solution, tonicity agent, pH adjusting agent, soothing agent and antiseptic, in distilled water for injection, subjecting the solution to disinfection filtration and placing the filtrate in an ample or vial, or freeze-drying the obtained filtrate to make a lyophilized dosage form. Examples of the additive include saccharides such as glucose, mannitol, xylitol and lactose; hydrophilic polymers such as polyethylene glycol; alcohols such as glycerol; amino acids such as glycine; proteins such as serum albumin; salts such as NaCl and sodium citrate; acids such as acetic acid, tartaric acid and ascorbic acid; surfactants such as Tween 80; reducing agents such as sodium sulfite; and the like. These formulations are used as injectable solutions or intravenous fluids by dissolving them in distilled water for injection or saline solution upon using them. Agents for intranasal administrations (transnasal administrations) such as a nasal drop and intranasal spray are also preferred for transmucosal administration, and an inhalant or the like is also preferred for transpulmonary administration.

It is preferred that the content of the active ingredient (such as desacyl ghrelin, the derivatives thereof, or the pharmacologically acceptable salts thereof) per unit formulation is 0.001 mg to 100 mg, preferably 0.01 mg to 10 mg, which is administered once or several times a day.

Isolated skin cells are treated by incubation of the isolated skin cells in a culture solution, followed by addition of 0.1 nM to 1 µM, preferably 1 nM to 100 nM, of desacyl ghrelin, the derivative thereof, or the pharmacologically acceptable salt thereof into the incubated solution prepared by disinfection filtration or autoclave disinfection. That is, it is preferred to use a culture solution containing 0.0000001 mg/mL to 0.1 mg/mL of the substance acting on GHS-R1a for proliferation of the skin cell. As shown in Example 2, this treatment accelerates proliferation of the skin cell, which hardly advances under normal conditions.

### Examples

Hereinafter, the present invention will be specifically described by way of Examples.

### (Example 1) Intracellular calcium increasing activity of desacyl ghrelin in cultured skin cell of rat fetus

A Wistar rat on the 17^{th} day of pregnancy underwent laparotomy under anesthesia for fetus extraction. A small piece of skin was collected from the fetus, treated with collagenase in cold Hank's solution, digested with papain, and mechanically separated by pipetting. This gave a dispersion liquid of the fetal skin cells. A single cell was obtained from the dispersion liquid, ghrelin was added thereto, and calcium imaging was performed. For the calcium imaging, a calcium imaging device (IMACS, Hamamatsu Photonics) was used. That is, the ratio of the emission at a wavelength of 510 nm was measured when excitation was performed by 340 nm/380 nm light. Fura-2 was used as a calcium imaging agent. Desacyl ghrelin derived from a rat (SEQ ID NO: 3) was used.

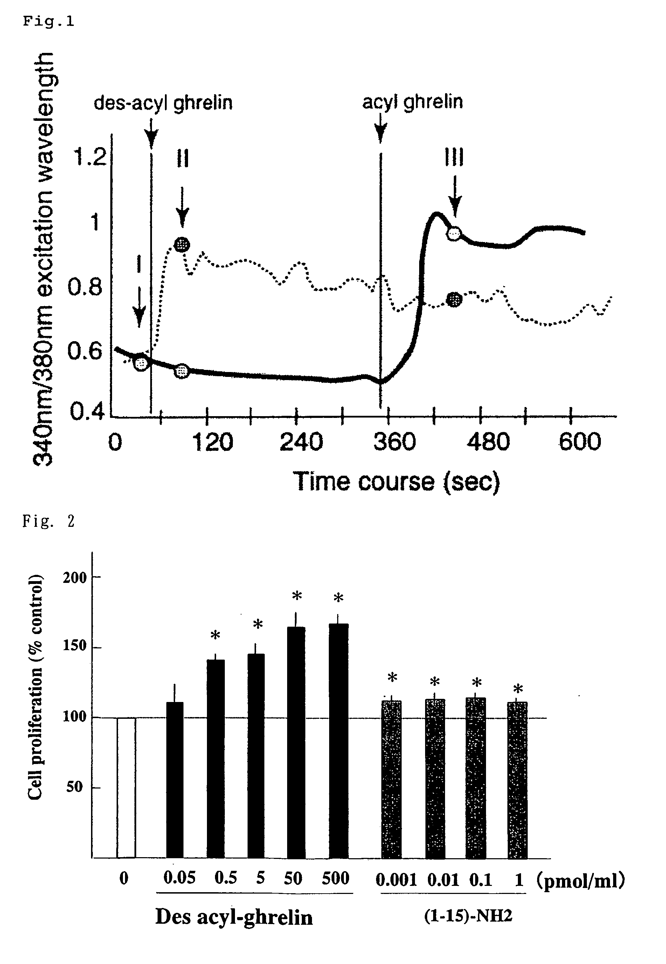

The results are shown in Fig. 1. In Fig. 1, I, II and III are the points at which a photograph was taken, but the photographs are omitted from the present description. The curve in full line shows a cell that responded to ghrelin, while the curve in dotted line shows another cell that responded to desacylghrelin. Ghrelin and desacylghrelin were added to different cells, respectively.

When desacyl ghrelin (1 × 10⁻⁶ M) acted on a skin cell of a single rat fetus, increase of intracellular calcium was observed. It was shown that a receptor that responds to desacyl ghrelin existed in the skin cell of the rat fetus, and that addition of desacyl ghrelin increased intracellular calcium, inducing the activity of desacyl ghrelin.

### (Example 2) Proliferation activity of desacyl ghrelin and desacyl ghrelin (1-15)-NH2 in cultured fetal skin cell

The skin cells of a rat fetus were collected from a Wistar rat on the 17^{th} day of pregnancy, and a dispersion liquid thereof was obtained in the same manner as in Example 1. The dispersed cells were suspended in a MCDB153HAA medium (F-Peptide Co., Ltd., Yamagata, Japan) containing 2% fetal bovine serum, penicillin (100 U/mL), streptomycin (100 µg/mL) and epidermal growth factor EGF (5 ng/mL), and sowed on a polyethyleneimine coated 96-hole multi-well plate in an amount of 3 × 10⁴ cells/well. Desacyl ghrelin (0.05 to 500 pmol/mL (nM)) or desacyl ghrelin (1-15)-NH2 (SEQ ID NO: 22) (0.001 to 1 pmol/mL (nM)) and 5-bromo-2'-deoxyuridine (BrdU) (10 µM) were added thereto, which was then incubated for 24 hours. A medium containing neither desacyl ghrelin nor desacyl ghrelin (1-15)-NH2 was used as a control. After the culture was over, cells were collected, and then uptake quantity of BrdU into the fetal skin cell was measured using Proliferation ELISA Kit (Roche Diagnostic GmbH. Manheim, Germany) to consider the skin cell growth activity.

The results are shown in Fig. 2.

Uptake of BrdU increased when desacyl ghrelin or desacyl ghrelin (1-15)-NH2 acted on a cultured fetal skin cell. Therefore, it was confirmed that desacyl ghrelin or desacyl ghrelin (1-15)-NH2 had an activity of allowing proliferation of a fetal skin cell.

The therapeutic agent for skin injuries, skin regeneration accelerator, growth acceleration method of a cultured skin cell, and skin regeneration acceleration method of the present invention can be applied in the pharmaceutical and medical fields.

## Claims

1. A therapeutic agent for skin injuries, comprising desacyl ghrelin or a peptide having physiological activity that is substantially identical to that of desacyl ghrelin, or a pharmaceutically acceptable salt thereof, as an active ingredient.

2. The therapeutic agent according to claim 1, wherein the active ingredient is a peptide selected from the group consisting of (i) a peptide having an amino acid sequence represented by SEQ ID NO: 1 to SEQ ID NO: 21, (ii) a peptide having the amino acid sequence from the N terminus to the 4^{th} amino acid residue of an amino acid sequence represented by SEQ ID NO: 1 to SEQ ID NO: 21, and having the amino acid sequence from the 5^{th} amino acid residue to the C terminus wherein one or several amino acids are deleted, substituted and/or added, (iii) peptides of (1) and (2), in which the C terminus is amidated, or a pharmaceutically acceptable salt thereof.

3. The therapeutic agent according to claim 2, wherein the active ingredient is a peptide having an amino acid sequence represented by SEQ ID NO: 1 or a pharmaceutically acceptable salt thereof.

4. The therapeutic agent according to claim 2, wherein the active ingredient is a peptide having the amino acid sequence from the N terminus to the 15^{th} amino acid residue of an amino acid sequence represented by SEQ ID NO: 1, of which the 15^{th} amino acid is amidated, or a pharmaceutically acceptable salt thereof.

5. The therapeutic agent according to any one of claims 1 to 4, including the active ingredient in an amount of 0.001 mg to 100 mg per unit formulation.

6. A skin regeneration accelerator, comprising desacyl ghrelin or a peptide having physiological activity that is substantially identical to that of desacyl ghrelin, or a pharmaceutically acceptable salt thereof, as an active ingredient.

7. The skin regeneration accelerator according to claim 6, wherein the active ingredient is a peptide selected from the group consisting of (i) a peptide having an amino acid sequence represented by SEQ ID NO: 1 to SEQ ID NO: 21, (ii) a peptide having the amino acid sequence from the N terminus to the 4^{th} amino acid residue of an amino acid sequence represented by SEQ ID NO: 1 to SEQ ID NO: 21, and having the amino acid sequence from the 5^{th} amino acid residue to the C terminus wherein one or several amino acids are deleted, substituted and/or added, (iii) peptides of (1) and (2), in which the C terminus is amidated, or a pharmaceutically acceptable salt thereof.

8. The skin regeneration accelerator according to claim 7, wherein the active ingredient is a peptide having an amino acid sequence represented by SEQ ID NO: 1 or a pharmaceutically acceptable salt thereof.

9. The skin regeneration accelerator according to claim 7, wherein the active ingredient is a peptide having the amino acid sequence from the N terminus to the 15^{th} amino acid residue of an amino acid sequence represented by SEQ ID NO: 1, of which the 15^{th} amino acid is amidated, or a pharmaceutically acceptable salt thereof.

10. The skin regeneration accelerator according to any one of claims 6 to 9, including the active ingredient in an amount of 0.001 mg to 100 mg per unit formulation.

11. A method for accelerating proliferation of a cultured skin cell, using desacyl ghrelin or a peptide having physiological activity that is substantially identical to that of desacyl ghrelin, or a pharmaceutically acceptable salt thereof, as an active ingredient.

12. The method according to claim 11, wherein the active ingredient is a peptide selected from the group consisting of (i) a peptide having an amino acid sequence represented by SEQ ID NO: 1 to SEQ ID NO: 21, (ii) a peptide having the amino acid sequence from the N terminus to the 4^{th} amino acid residue of an amino acid sequence represented by SEQ ID NO: 1 to SEQ ID NO: 21, and having the amino acid sequence from the 5^{th} amino acid residue to the C terminus wherein one or several amino acids are deleted, substituted and/or added, (iii) peptides of (1) and (2), in which the C terminus is amidated, or a pharmaceutically acceptable salt thereof.

13. The method according to claim 12, wherein the active ingredient is a peptide having an amino acid sequence represented by SEQ ID NO: 1 or a pharmaceutically acceptable salt thereof.

14. The method according to claim 12, wherein the active ingredient is a peptide having the amino acid sequence from the N terminus to the 15^{th} amino acid residue of an amino acid sequence represented by SEQ ID NO: 1, of which the 15^{th} amino acid is amidated, or a pharmaceutically acceptable salt thereof.

15. The method according to any one of claims 11 to 14, wherein proliferation of the cultured skin cell is performed in a medium containing the active ingredient in an amount of 0.0000001 mg/mL to 0.1 mg/mL.

16. A treatment method of skin injuries, comprising administration of desacyl ghrelin or a peptide having physiological activity that is substantially identical to that of desacyl ghrelin, or a pharmaceutically acceptable salt thereof as an active ingredient to a mammal requiring treatment of skin injuries.

17. The method according to claim 16, wherein the active ingredient is a peptide selected from the group consisting of (i) a peptide having an amino acid sequence represented by SEQ ID NO: 1 to SEQ ID NO: 21, (ii) a peptide having the amino acid sequence from the N terminus to the 4^{th} amino acid residue of an amino acid sequence represented by SEQ ID NO: 1 to SEQ ID NO: 21, and having the amino acid sequence from the 5^{th} amino acid residue to the C terminus wherein one or several amino acids are deleted, substituted and/or added, (iii) peptides of (1) and (2), in which the C terminus is amidated, or a pharmaceutically acceptable salt thereof.

18. The method according to claim 17, wherein the active ingredient is a peptide having an amino acid sequence represented by SEQ ID NO: 1 or a pharmaceutically acceptable salt thereof.

19. The method according to claim 17, wherein the active ingredient is a peptide having the amino acid sequence from the N terminus to the 15^{th} amino acid residue of an amino acid sequence represented by SEQ ID NO: 1, of which the 15^{th} amino acid is amidated, or a pharmaceutically acceptable salt thereof.

20. The method according to any one of claims 16 to 19, wherein the active ingredient is administered in an amount of 0.001 mg to 100 mg per administration.

21. A method for accelerating skin regeneration, comprising administration of desacyl ghrelin or a peptide having physiological activity that is substantially identical to that of desacyl ghrelin, or a pharmaceutically acceptable salt thereof as an active ingredient to a mammal requiring skin regeneration.

22. The method according to claim 21, wherein the active ingredient is a peptide selected from the group consisting of (i) a peptide having an amino acid sequence represented by SEQ ID NO: 1 to SEQ ID NO: 21, (ii) a peptide having the amino acid sequence from the N terminus to the 4^{th} amino acid residue of an amino acid sequence represented by SEQ ID NO: 1 to SEQ ID NO: 21, and having the amino acid sequence from the 5^{th} amino acid residue to the C terminus wherein one or several amino acids are deleted, substituted and/or added, (iii) peptides of (1) and (2), in which the C terminus is amidated, or a pharmaceutically acceptable salt thereof.

23. The method according to claim 22, wherein the active ingredient is a peptide having an amino acid sequence represented by SEQ ID NO: 1 or a pharmaceutically acceptable salt thereof.

24. The method according to claim 22, wherein the active ingredient is a peptide having the amino acid sequence from the N terminus to the 15^{th} amino acid residue of an amino acid sequence represented by SEQ ID NO: 1, of which the 15^{th} amino acid is amidated, or a pharmaceutically acceptable salt thereof.

25. The method according to any one of claims 21 to 24, wherein the active ingredient is administered in an amount of 0.001 mg to 100 mg per administration.

26. Use of desacyl ghrelin or a peptide having physiological activity that is substantially identical to that of desacyl ghrelin, or a pharmaceutically acceptable salt thereof as an active ingredient for production of a therapeutic agent for skin injuries.

27. The use according to claim 26, wherein the active ingredient is a peptide selected from the group consisting of (i) a peptide having an amino acid sequence represented by SEQ ID NO: 1 to SEQ ID NO: 21, (ii) a peptide having the amino acid sequence from the N terminus to the 4^{th} amino acid residue of an amino acid sequence represented by SEQ ID NO: 1 to SEQ ID NO: 21, and having the amino acid sequence from the 5^{th} amino acid residue to the C terminus wherein one or several amino acids are deleted, substituted and/or added, (iii) peptides of (1) and (2), in which the C terminus is amidated, or a pharmaceutically acceptable salt thereof.

28. The use according to claim 27, wherein the active ingredient is a peptide having an amino acid sequence represented by SEQ ID NO: 1 or a pharmaceutically acceptable salt thereof.

29. The use according to claim 27, wherein the active ingredient is a peptide having the amino acid sequence from the N terminus to the 15^{th} amino acid residue of an amino acid sequence represented by SEQ ID NO: 1, of which the 15^{th} amino acid is amidated, or a pharmaceutically acceptable salt thereof.

30. The use according to any one of claims 26 to 29, wherein the therapeutic agent for skin injuries contains the active ingredient in an amount of 0.001 mg to 100 mg per unit formulation.

31. Use of desacyl ghrelin, or a peptide having a physiological action that is substantially identical to that of desacyl ghrelin, or a pharmaceutically acceptable salt thereof as an active ingredient for production of a skin regeneration accelerator.

32. The use according to claim 31, wherein the active ingredient is a peptide selected from the group consisting of (i) a peptide having an amino acid sequence represented by SEQ ID NO: 1 to SEQ ID NO: 21, (ii) a peptide having the amino acid sequence from the N terminus to the 4^{th} amino acid residue of an amino acid sequence represented by SEQ ID NO: 1 to SEQ ID NO: 21, and having the amino acid sequence from the 5^{th} amino acid residue to the C terminus wherein one or several amino acids are deleted, substituted and/or added, (iii) peptides of (1) and (2), in which the C terminus is amidated, or a pharmaceutically acceptable salt thereof.

33. The use according to claim 32, wherein the active ingredient is a peptide having an amino acid sequence represented by SEQ ID NO: 1 or a pharmaceutically acceptable salt thereof.

34. The use according to claim 32, wherein the active ingredient is a peptide having the amino acid sequence from the N terminus to the 15^{th} amino acid residue of an amino acid sequence represented by SEQ ID NO: 1, of which the 15^{th} amino acid is amidated, or a pharmaceutically acceptable salt thereof.

35. The use according to any one of claims 31 to 34, wherein the skin regeneration accelerator contains the active ingredient in an amount of 0.001 mg to 100 mg per unit formulation.
